# EUROPEAN PATENT APPLICATION

(11) **EP 2 458 519 A1**
(43) Date of publication of application: **30.05.2012**
(21) Application number: 11189990.2
(22) Date of filing: 21.11.2011
(51) Int. Cl.: G06F 19/00

(54) **Method and apparatus of informing about a sensor mounting time period**

(30) Priority: 25.11.2010 JP 2010262486
(71) Applicant: Nihon Kohden Corporation, Shinjuku-ku Tokyo 161-8560 (JP)
(72) Inventor: Kobayashi, Naoki, Tokyo, 161-8560 (JP); Takahashi, Iwao, Tokyo, 161-8560 (JP); Shindo, Yoshiaki, Tokyo, 161-8560 (JP); Ito, Kazumasa, Tokyo, 161-8560 (JP)
(74) Representative: 2K Patentanwälte Blasberg Kewitz & Reichel

(57) **Abstract**

The sensor mounting time period informing apparatus 200 is provided with the detecting part 100, the timer part 120, the discriminating part 130, and the informing part 140. The detecting part detects whether or not the sensor 20 is mounted on the living body 10 for measuring biological information. The timer part 120 counts the elapsed time period during which the sensor 20 is continuously mounted on the living body 10 based on the detection results of the detecting part 100. The discriminating part 130 discriminates whether or not the elapsed time has reached the specified standard time. The informing part 140 issues a notification when the elapsed time has reached the standard time based on the discrimination result of the discriminating part 130.

## Description

### CROSS-REFERENCE TO RELATED APPLICATION

This application is based on Japanese Patent Application No. 2010-262486 filed on November 25, 2010, the contents of which are incorporated herein by reference.

### BACKGROUND

### 1. Technical Field

The present invention relates to a method of informing about a sensor mounting time period and an apparatus for informing about a sensor mounting time period.

### 2. Description of Related Arts

In clinical practice, it is required to monitor patient's biological information such as pulse and blood pressure continuously in a non-invasive manner. The current status of medical institutions such as hospitals is that patient's biological information is measured continuously using medical devices called biological information monitors in order to monitor patients' physical conditions. A biological information monitor collects biological information of a patient using sensors attached to the patient, processes the biological information as the information related to the patient's physical conditions, and converts it into a format including numerical values and the like, before transmitting the information to medical professionals such as doctors and nurses.

In general, the sensors of a biological information monitor are mounted on the patient's body under closely contacting conditions from the standpoint of maintaining best measurement accuracies at all times. On the other hand, maintaining a sensor in close contact for a prolonged period may cause pain due to pressure or a rash in the area of contact. As a way of mitigating such a burden associated with the sensor mounting by improving the sensor's construction, a technology for mitigating the patient's burden by making the sensor more compact and lighter is disclosed in Japanese Unexamined Publication No. 2007-54594. Also, Japanese Unexamined Publication No. 2008-245701 disclose a technology of mitigating the burden caused by a localized pressure on a patient by applying a more uniform pressure in mounting a sensor on the patient's body.

However, both technologies disclosed by Japanese Unexamined Publication No. 2007-54594 and Japanese Unexamined Publication No. 2008-245701 rely on the premise that the sensor mounting positions are changed periodically with a specified time interval, while whether the sensor mounting positions are actually changed or not within the specified time interval depends on how seriously the medical professionals who are in charge of handling the biological information monitor recognize the need for changing the mounting positions. Therefore, if the sensor mounting positions are not changed within the specified time period, the patient's burden may increase due to the continued mounting of the sensors.

The present invention was made to solve the problems shown above. The object of the present invention is to provide a method and an apparatus for informing to medical professionals the elapsed time period during which a sensor communicating with the apparatus is mounted continuously on the patient's body, so that the sensor's mounting position is changed within the specified time interval.

### SUMMARY

In order to achieve at least one of the aforementioned objects, the method of informing the sensor mounting time period according to the present invention is to measure the time a sensor has been mounted on the living body for measuring biological information and issues a notification when the time reached a specified standard time.

Moreover, the sensor mounting time period informing apparatus according to the present invention is provided with detecting part, timer part, discriminating part, and informing part. The detecting part detects whether or not any sensor, communicating with the apparatus, is mounted on the living body for measuring biological information. The timer part counts the elapsed time period during which the sensor is continuously mounted on the living body based on the detection result of the detecting part. The discriminating part discriminates whether or not the elapsed time has reached the specified standard time. The informing part issues a notification when the elapsed time has reached the standard time based on the discrimination result of the discriminating part.

The objects, features, and characteristics of this invention other than those set forth above will become apparent from the description given herein below with reference to preferred embodiments illustrated in the accompanying drawings.

### BRIEF DESCRIPTION OF THE DRAWINGS

Fig. 1 shows an outline block diagram for describing the apparatus for informing about a sensor mounting time period according to a first embodiment of the present invention.
Fig. 2 is a block diagram for describing the detecting part shown in Fig. 1.
Fig. 3 is a waveform diagram showing a chronological change of transmitted light signals emitted from the demodulation part shown in Fig. 2.
Fig. 4 shows a flowchart for describing the method of informing about a sensor mounting time period according to the first embodiment of the present invention.
Fig. 5 shows an outline block diagram for describing the apparatus for informing about a sensor mounting time period according to a second embodiment of the present invention.

### DETAILED DESCRIPTION

Embodiments of the apparatus for informing about a sensor mounting time period will be described below with reference to the accompanying drawings. Same symbols are applied to identical members found in various drawings. The scaling factors of the drawings may vary from those of the actual components because of intentional exaggerations for the sake of explanations.

### (First embodiment)

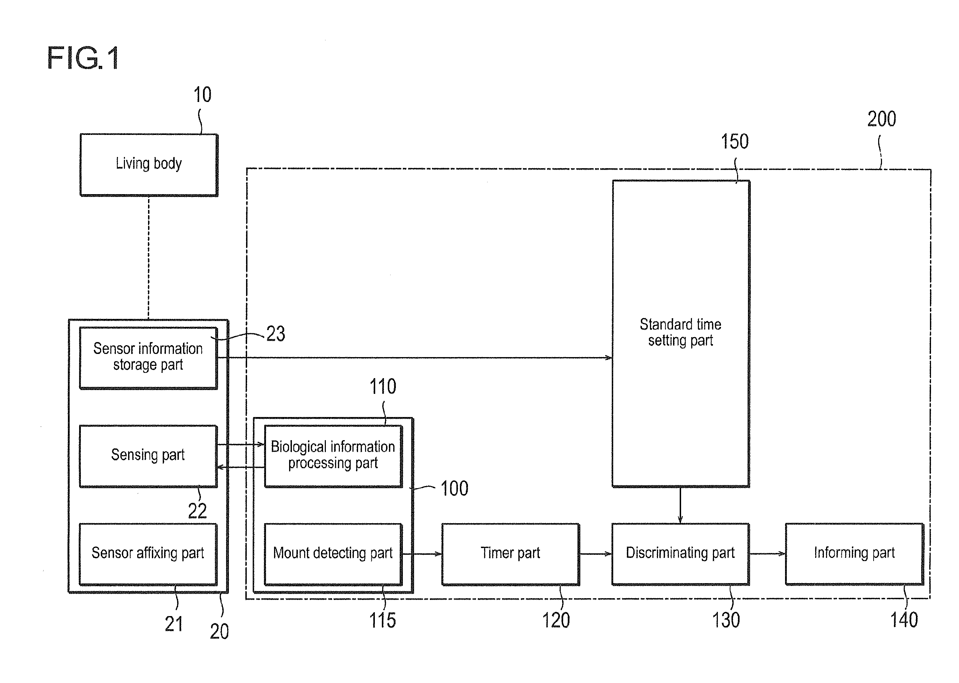

Fig. 1 shows an outline block diagram for describing the apparatus for informing about a sensor mounting time period according to a first embodiment. In Fig. 1, the apparatus for informing about a sensor mounting time period is shown by the range surrounded by the chain line. The apparatus for informing about a sensor mounting time period is to notify that the duration of the time when a biological information sensor, which communicates with the apparatus, is mounted on the living body including patient's body, continuously has reached a specified standard time using the biological information measured by the sensor of the biological information monitor.

The present embodiment will be described based mainly on a case of informing the sensor mounting time period of the pulse oximeter used as a biological information monitor. The pulse oximeter is a device for measuring oxygen saturation (SₚO₂) of arterial blood continuously in a non-invasive manner. However, the biological information monitor herein is not limited to the pulse oximeter, but rather it is possible to constitute an apparatus for informing about a sensor mounting time period so long as the sensor of the biological information monitor other than the pulse oximeter is of a type that is mounted on the surface of a living body. Examples of applications to biological information monitors other than the pulse oximeter will be described later.

As shown in Fig. 1, an apparatus for informing about a sensor mounting time period 200 according to the present embodiment has a detecting part 100, a timer part 120, an discriminating part 130, an informing part 140, and a standard time setting part 150. The detecting part 100 has a biological information processing part 110 and a mount detecting part 115, and is connected to a sensor 20 by a cable or by a wireless device. Fig. 1 shows the relation between a living body 10 and the sensor 20 that detects the living body 10 by dotted lines.

The sensor 20 is mounted on the living body 10 to measure biological information. More specifically, the sensor of the pulse oximeter, normally called a "probe" is mounted on a part of the surface of the living body (e.g., finger tip or forehead of the patient) to measure the transmitted light (transmission type probe) or reflected light (reflection type probe) of the light irradiated on the living body 10. The sensor 20 has a sensor affixing part 21, a sensing part 22, and a sensor information storage part 23.

The sensor affixing part 21 affixes the sensing part 22 to the surface of the living body when mounting the sensor 20 on the living body 10. More specifically, the sensor affixing part 21 affixes the sensing part 22 in such a way to have the light emitting part and the light receiving part of the sensing part 22 placed in serial via the living body 10 in case of a transmission type probe. Also, it affixes the sensing part 22 in such a way as to have the illuminating part and the light receiving part of the sensing part 22 placed in parallel with the patient's body in case of a reflection type probe.

The sensor affixing part 21 has various affixing devices including a clip type affixing device to pinch the patient's finger, an adhesive tape type affixing device to apply an adhesive tape on a patient's finger, a type which looks like a finger stall to be capped on a finger, etc., for the transmission type probe, and an adhesive type affixing device to be applied on the patient's forehead, etc., for the reflection type probe. As there are so many kinds of sensor affixing part 21, how long the sensor 20 can stay mounted on the living body 10 continuously depends on what kind of sensor affixing part 21 is used. In the following explanation, a case of using a transmission type probe is used as the sensor 20.

The sensing part 22 irradiates the living body 10 with a red light and an infrared light and receives the transmitted lights.

The sensing part 22 is equipped with a light emitting part and a light receiving part, and is connected to the biological information processing part 110 of the detecting part 100. The light emitting part is equipped with a light emitting diode that emits lights with wave lengths in the neighborhood of 660 nm (red) and also 940 nm (infrared) and emits the lights onto a part of the surface of the living body such as finger tip. The light receiving part is equipped with, for example, a photo-diode to receive the light transmitted through blood vessels and living organisms, converts it to an electric current signal, and outputs it to the detecting part 100.

The sensor information storage part 23 stores the type of the sensor 20. More specifically, the sensor information storage part 23 stores the information concerning the sensor 20 including the type of the sensor affixing part 21 and others in a memory element such as a resistor or a memory (ROM). The memory element is, for example, embedded in a sensor-side connector of the cable that connects the sensor 20 with the detecting part 100.

The apparatus for informing about a sensor mounting time period 200 reads the information concerning the type of the sensor 20 stored in the sensor information storage part 23, and transmits it to the standard time setting part 150.

The detecting part 100 detects whether the sensor 20 is mounted on the living body 10 or not. The detecting part 100 has a biological information processing part 110 and a mount detecting part 115, and is connected to the sensor 20 and the timer part 120.

The biological information processing part 110 measures biological information to transmit it to the mount detecting part 115, and also processes the biological information to calculate the oxygen saturation of arterial blood. The mount detecting part 115 detects whether or not the sensor 20 is mounted on the living body 10 based on the biological information measured.

Since biological information is information obtained biologically from the living body, the fact that biological information is obtained by the biological information measuring part 110 means that the sensor 20 is mounted on the living body 10.

The mount detecting part 115 generates a count-enable signal for clock operations and transmits it to the timer part 120. When the sensor 20 is detected as being mounted on the living body 10, the mount detecting part 115 activates the clock-enabling signal. When the sensor 20 is detected as not being mounted on the living body 10, the mount detecting part 115 does not activate the clock-enabling signal. The specific configurations of the biological information processing part 110 and the mount detecting part 115 will be described later.

The timer part 120 counts the elapsed time period when the sensor 20 is continuously mounted on the living body 10 based on the detection result of the detecting part 100. The timer part 120 is connected to the detecting part 100 and the discriminating part 130.

The timer part 120 starts timer as it receives the clock-enabling signal from the detecting part 100 and measures the time period during which the sensor 20 is continuously mounted on the living body 10. The timer part 120 is equipped with, for example, a digital counter that operates with a specified clock cycle, and starts counting time with the digital counter as it receives the clock-enabling signal. The timer part 120 transmits the duration time period that corresponds with the count value to the discriminating part 130.

The timer part 120 continues the counting operation even during a period when the mounted sensor 20 is temporarily (e.g., less than 1 second) is removed from the living body 10 and the biological information is not measured temporarily. In other words, it treats the temporary removal of the sensor 20 from the living body 10 as if the sensor 20 is continuously mounted on the living body 10. On the other hand, if it is judged that the removal of the sensor 20 is not temporary, it resets the digital counter and waits for the sensor 20 to be mounted again on the living body 10.

The timer can also be started when a medical professional operates a timer start key (not shown). Consequently, the mounting start time of the sensor 20 can be more securely determined.

The discriminating part 130 discriminates whether or not the elapsed time period during which the sensor 20 is mounted continuously on the living body 10 has reached the specified standard time. The discriminating part 130 is connected to the timer part 120 and the informing part 140.

The discriminating part 130 generates the standard time reaching signal for informing whether or not the elapsed time period has reached the standard time and transmits it to the informing part 140. The discriminating part 130 activates the standard time reaching signal when the elapsed time period received from the timer part 120 reaches the standard time. On the other hand, the discriminating part 130 does not activate the standard time reaching signal when the elapsed time period has not reached the standard time.

The informing part 140 issues a notification based on the discrimination result of the discriminating part 130 when the elapsed time has reached the standard time. More specifically, the informing part 140 is connected to the discriminating part 130 and executes the informing action upon receiving the standard time reaching signal from the discriminating part 130.

The informing part 140 is equipped with, e.g., a speaker, lamp, monitor display, etc., and is capable of informing by an alarm sound, voice message, light, optical message, etc., upon receiving the standard time reaching signal from the discriminating part 130. Moreover, the informing part 140 is equipped with a communication device so that it is also capable of informing a medical professional at a central control center located at a distance from the patient's current position.

The standard time setting part 150 is connected to the sensor information storage part 23 and the discriminating part 130, generates the standard time and transmits it to the discriminating part 130. The standard time is the time determined depending on the type of the sensor 20 based on the time period during which the sensor 20 can be mounted at the same location of the surface of the living body continuously.

The type of the sensor 20 is reported to the standard time setting part 150 by the sensor information storage part 23 of the sensor 20. The type of the sensor 20 depends on, e.g., the type of the sensor affixing part 21. The standard time setting part 150 can set the standard time depending on the type of the sensor affixing part 21. The standard time setting part 150 sets, for example, the standard time as 4 hours when the sensor affixing part 21 is a clip-style affixing device, or 8 hours when the sensor affixing part 21 is an adhesive-type affixing device. The setting of the standard time depending on the type of the sensor affixing part 21 can be arbitrarily changed.

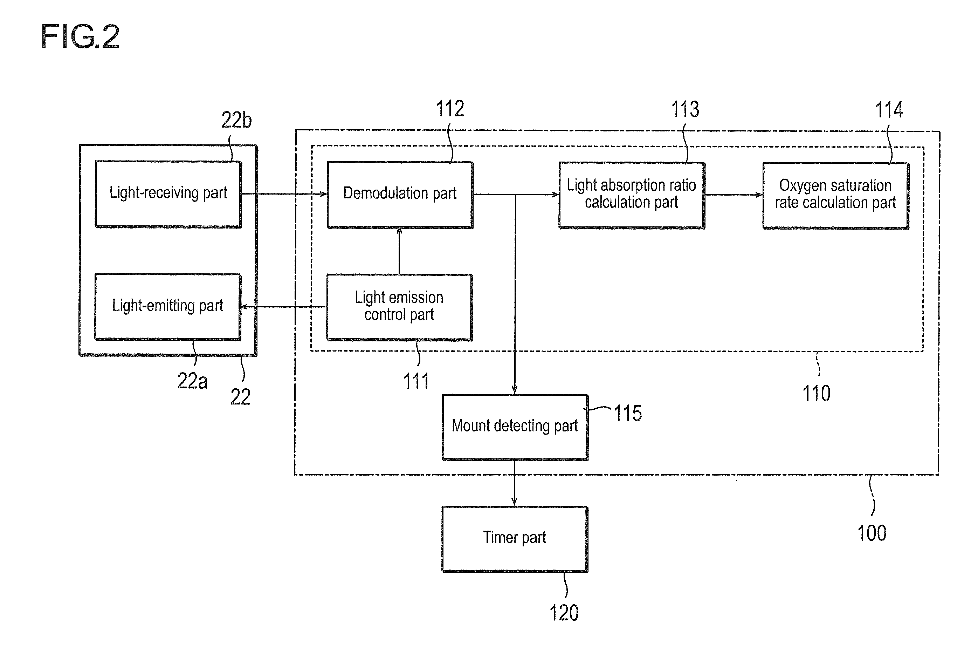

Next, the detecting part shown in Fig. 1 will be described in further details with reference to Fig. 2. As shown in Fig. 2, the detecting part 100 has a biological information processing part 110 and a mount detecting part 115. The biological information processing part 110 has a light emission control part 111, a demodulation part 112, light absorbance ratio calculation part 113, and an oxygen saturation calculation part 114, transmits biological information to the mount detecting part 115, and calculates the oxygen saturation of arterial blood by processing the biological information.

The light emission control part 111 controls the timing of illumination of a light emitting part 22a of the sensing part 22. More specifically, the light emission control part 111 generates a light emission timing signal and controls it in such a manner that a red light-emitting diode and a infrared light-emitting diode illuminates reciprocally at a given timing.

The modulation part 112 generates red transmitted light signals and infrared transmitted light signals. More specifically, the demodulation part 112 converts electric current signals generated by a light-receiving part 22b of the sensing part 22 into electric voltage signals, and then generates red transmitted light signals and infrared transmitted light signals by demodulating the electric voltage signals using the light emission timing signals generated by the light emission control part 111.

The red lights and the infrared lights entering the living body 10 from the light-emitting part 22a are received by the light-receiving part 22b after passing through body tissues, venous blood and arterial blood. The transmitted light signal weakens when the blood flow volume in the artery increases as the patient's heart contracts, while the transmitted light signal strengthens when the blood flow volume in the artery reduces as the heart expands. Therefore, the transmitted light varies with the variation of the blood flow volume in the artery due to the pulsation of the heart.

The light absorbance ratio calculation part 113 calculates the ratio between the light absorbance of the red light and the infrared light. The transmitted light signal generated in the demodulation part 112 appears in a wavy form in which an AC component (pulsation component) is superimposed on a DC component. The light absorbance ratio calculation part 113 separates the DC component and the AC component for each of the red transmitted light signal and the infrared transmitted signal and calculates pulsation ratios (AC component/DC component). The light absorbance ratio calculation part 113 calculates an approximate ratio between the absorbances of the red light and the infrared light by calculating the ratio of the pulsation rate of the red light and the pulsation rate of the infrared light.

The oxygen saturation calculation part 114 calculates the arterial blood oxygen saturation of hemoglobin from the ratio between the red light and the infrared light. As to the process of this calculation, it is similar to the calculation process in the pulse oximeter of the prior art so that detailed explanation is omitted here. The calculated arterial blood oxygen saturation is displayed on a display device not shown here.

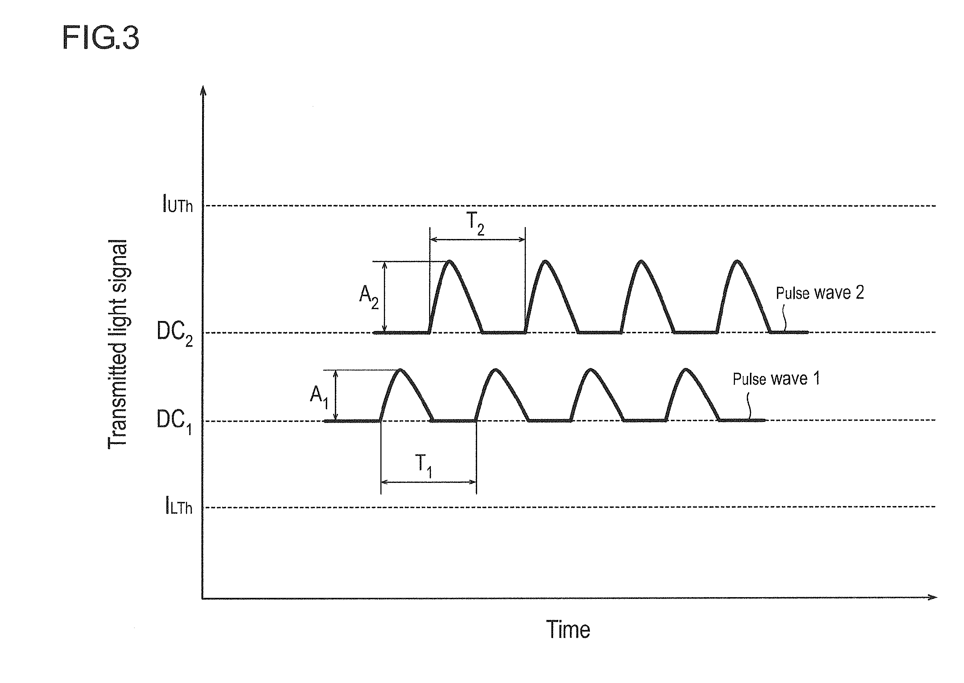

The mount detecting part 115 detects whether or not the sensor 20 is mounted on the living body based on the biological information signal received from the biological information processing part 110. More specifically, the mount detecting part 115 is connected to the demodulation part 112 of the biological information processing part 110, and detects whether or not the sensor 20 is mounted on the living body 10 using the transmitted light signal generated by the demodulation part 112. The method of detecting whether or not the sensor 20 is mounted on the living body 10 using the transmitted light signal is described below with reference to Fig. 3.

Fig. 3 is a waveform diagram showing a chronological change of transmitted light signals emitted from the demodulation part 112 shown in Fig. 2. The vertical axis of Fig. 3 represents the intensity of the transmitted light signal and the horizontal axis represents the elapsed time.

Fig. 3 shows the transmitted light signal outputted from the demodulation part 112 when the living body 10 is irradiated with red light and infrared light, and the transmitted light signal reflect the pulse waves of the red light and the infrared light. In the following, the pulse waves measured for the red light and the infrared light will be denoted as the pulse wave 1 and the pulse wave 2. Incidentally, in Fig. 3, the DC component DC₂ of the transmitted light signal of the pulse wave 2 is shown larger than the maximum valued of the transmitted light signal of the pulse wave 1 for the convenience of drawing the pulse wave 1 and the pulse wave 2.

The DC components DC₁ and DC₂ of the transmitted light signals of the pulse wave 1 and the pulse wave 2 depend on the thickness of body tissue and the amount of venous blood, while the amplitudes A1 and A2 depend on the degree of combination between hemoglobin and oxygen in the arterial blood.

When the sensor 20 of the pulse oximeter is mounted properly on the specified position of the living body 10, the DC components DC₁ and DC₂ of the pulse wave 1 and the pulse wave 2, the amplitudes A₁ and A₂, and the intervals T₁ and T₂ assume values within their respective specified ranges. The specified position of the living body 10 here means the part of a relatively thin tissue of the living body 10, for example, the finger tip.

As shown in Fig. 3, when the DC components DC₁ and DC₂ assumes the values between the upper threshold value I_{UTh} and the lower threshold I_{LTh} of the transmitted light signal intensity, the DC components DC₁ and DC₂ lie within the specified ranged. On the other hand, if the DC components DC₁ and DC₂ do not assume the values between the upper threshold value I_{UTh} and the lower threshold I_{LTh}, the DC components DC₁ and DC₂ do not lie within the specified ranged. In this case, there is a probability that the sensor 20 may not be properly mounted at the specified position of the living body 10.

For example, if the DC components DC₁ or DC₂ is greater than the upper threshold value I_{UTh}, there is a possibility that the finger tip is not properly inserted between the light-emitting part 22a and the light-receiving part 22b of the sensor 20, so that the light from the light-emitting part may be directly transmitted to the light-receiving part 22b.

On the other hand, if the DC components DC₁ or DC₂ is smaller than the lower threshold I_{LTh}, there is a possibility that the sensor 20 is removed from the finger tip, or the sensor 20 is mounted on a living body part thicker than the finger tip, so that the light may not be transmitted between the light-emitting part 22a and the light-receiving part 22b of the sensor 20.

Moreover, the amplitudes A₁ and A₂ of the pulse wave 1 and the pulse wave 2 assume values within the specified range depending on the degree of combination between hemoglobin and oxygen within arterial blood. The specified range herein can be arbitrarily determined based on the degree of combination between hemoglobin and oxygen and the relation between the transmitted red light and infrared light of the living body. When the amplitudes A₁ and A₂ are not values within the specified range, there is a possibility that the sensor 20 may not be properly mounted at the specified position of the living body 10.

Moreover, the intervals T₁ and T₂ of the pulse wave 1 and the pulse wave 2 may assume values within the specified range in correspondence with the change of the volume of the arterial blood. When the intervals T₁ and T₂ are not values within the specified range, there is a possibility that the sensor 20 may not be properly mounted at the specified position of the living body 10.

Therefore, it is possible to make a judgment as to whether or not the sensor 20 is properly mounted at the specified position of the living body 10 based on whether or not the DC components DC₁ and DC₂, the amplitudes A₁ and A₂, and the intervals T₁ and T₂ are the values within the specified ranges respectively.

Moreover, in addition to the DC components, amplitudes and intervals, other items such as the rising time or shape of the pulse wave can be added as the judgment conditions. Furthermore, the ratio between the transmitted light and the incident light can be used instead of the absolute value of the transmitted light as the transmitted light signal.

As can be seen from the above, whether or not the sensor 20 is mounted on the living body 10 is first detected in the apparatus 200 for informing about a sensor mounting time period according to the present embodiment. More specifically, when the sensor 20 is detected as being mounted on the living body 10, the detecting part 100 activates the clock-enabling signal. On the other hand, when the sensor 20 is detected as not being mounted on the living body 10, the detecting part 100 does not activate the clock-enabling signal.

Next, the elapsed time period during which the sensor 20 is mounted continuously on the living body 10 is measured based on the detection result of whether the sensor 20 is mounted on the living body 10. More specifically, the timer part 120 starts timer as it receives the clock-enabling signal from the detecting part 100 and measures the elapsed time period during which the sensor 20 is continuously mounted on the living body 10.

Next, it is judged whether or not the elapsed time has reached the specified standard time. More specifically, the discriminating part 130 activates the standard time reaching signal when the elapsed time period received from the timer part 120 reaches the standard time. On the other hand, the discriminating part 130 does not activate the standard time reaching signal when the elapsed time period has not reached the standard time.

Next, a notification that the elapsed time has reached the standard time is issued based on discrimination result of the discriminating part 130. More specifically, the informing part 140 receives the standard time reaching signal from the discriminating part 130 and notifies medical professionals with an alarm sound, voice, light or visual message.

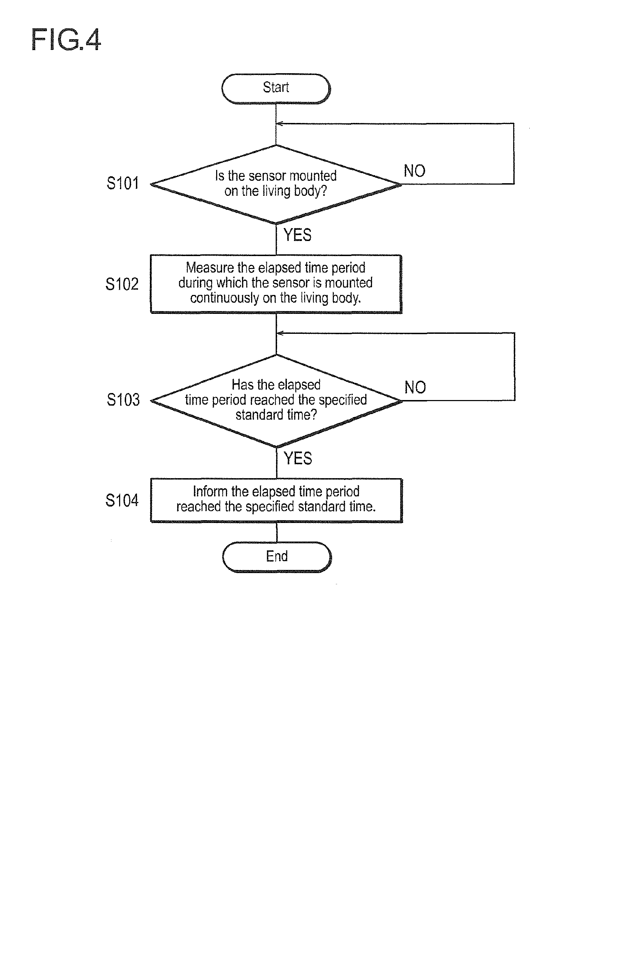

Such is the configuration of the apparatus 200 for informing about a sensor mounting time period according to the present embodiment. The method of informing about a sensor mounting time period according to the present embodiment will be described below with reference to Fig. 4. Fig. 4 shows a flowchart for describing the method of informing about a sensor mounting time period according to the embodiment. The method of informing the sensor mounting time period is to measure the time a sensor has been mounted continuously on the living body for measuring biological information and issues a notification when the time reached a specified standard time.

As shown in Fig. 4, a judgment is made as to whether or not the sensor communicating with the apparatus is mounted on the living body (step S101). If it is confirmed that the sensor communicating with the apparatus is mounted on the living body (steps S101: YES), the program advances to the next step S102. On the other hand, if it is not confirmed that the sensor communicating with the apparatus is mounted on the living body (steps S101: NO), the program awaits for the sensor to be mounted on the living body.

Next, the elapsed time period during which a sensor communicating with the apparatus is mounted continuously on the living body is measured (step S102).

Next, a judgment is made as to whether or not the elapsed time has reached the specified standard time (step S103). When the elapsed time period reaches the standard time (step S 103: YES), the program advances to the next step S 104. On the other hand, if the elapsed time period does not reach the specified standard time (step S103: NO), the program awaits until the elapsed time reaches the specified standard time.

So far, the configuration of the pulse oximeter as the biological information monitor for informing the sensor mounting time period has been described. Next, the configurations of biological information monitors other than the pulse oximeter for informing the sensor mounting time period will be described below. The apparatuses for informing about a sensor mounting time period described below are different from the apparatus for informing about a sensor mounting time period based on the pulse oximeter only in the configurations of the detecting part and the sensor. Therefore, only the configurations of the detecting part and the sensor are explained and the configurations of other parts will be omitted in the following descriptions.

### (Electrocardiograph)

The apparatus for informing about a sensor mounting time period using the electrocardiograph uses a plurality of electrodes in its sensing part of the sensor. The electrodes are affixed to the living body with the sensor affixing part including adhesive tapes and the cardiogram signals from the electrodes are transmitted to the biological information processing part of the detecting part.

The biological information processing part amplifies the cardiograph signals, extracts cardiogram waveforms, and transmits them to the mount detecting part. As to the part that measures and processes the cardiogram waveforms in the biological information processing part is similar to that of the conventional cardiograph, so that its description is omitted here. The mount detecting part measures the cardiogram waveforms extracted by the biological information processing part to obtain amplitudes and intervals, determines whether or not the sensor communicating with the apparatus is mounted on the living body based on whether or not the measured values are within the specified ranges. Moreover, the shapes of the cardiogram waveforms can be used as an additional condition in the judgment as well.

### (Electroencephalograph)

The apparatus for informing about a sensor mounting time period using the electroencephalograph uses a plurality of electrodes in its sensing part of the sensor. The electrodes are typically made as needle electrodes or dish electrodes and are affixed by the sensor affixing part including adhesive tapes or cap-lie affixing equipment. The electroencephalogram signals from the electrodes are transmitted to the biological information processing part of the detecting part.

The biological information processing part amplifies the electroencephalogram signals, extracts electroencephalogram waveforms, and transmits them to the mount detecting part. As to the part that measures and processes the electroencephalogram waveforms in the biological information processing part is similar to that of the conventional electroencephalograph, so that its description is omitted here. The mount detecting part measures the electroencephalogram waveforms extracted by the biological information processing part to obtain amplitudes and intervals, determines whether or not the sensor communicating with the apparatus is mounted on the living body based on whether or not the measured values are within the specified ranges. Moreover, the rising times and shapes of the electroencephalogram waveforms can be used as additional conditions in the judgment as well.

### (Clinical thermometer)

The apparatus for informing about a sensor mounting time period using the clinical thermometer uses a thermister in its sensing part of the sensor. The thermister is affixed to the living body with the sensor affixing part including adhesive tapes and the body temperature from the thermister is transmitted after converting it an electrical signal to the biological information processing part of the detecting part.

The biological information processing part measures the body temperature and transmits it to the mount detecting part. As to the part that measures and processes the body temperature in the biological information processing part is similar to that of the conventional clinical thermometer, so that its description is omitted here. The mount detecting part detects whether or not the sensor communicating with the apparatus is mounted on the living body based on the measured body temperature is within the specified range.

### (Phonocardiograph)

The apparatus for informing about a sensor mounting time period using the phonocardiograph uses a microphone in its sensing part of the sensor. The microphone is affixed to the living body with the sensor affixing part including adhesive tapes and the phonocardiogram is transmitted after converting it an electrical signal to the biological information processing part of the detecting part.

The biological information processing part, after amplifying and wave-filtering the transmitted electrical signal, extracts only the sound from the heart, and transmits it as the phonocardiogram signal to the mount detecting part. As to the part that measures and processes the phonocardiogram in the biological information processing part is similar to that of the conventional phonocardiograph, so that its description is omitted here. The mount detecting part measures the phonocardiogram signal extracted by the biological information processing part to obtain DC components, amplitudes and intervals, determines whether or not the sensor communicating with the apparatus is mounted on the living body based on whether or not the measured values are within the specified ranges. Moreover, the rising times and shapes of the phonocardiogram signals can be used as additional conditions in the judgment as well.

### (Percutaneous gas monitor)

The apparatus for informing about a sensor mounting time period using the percutaneous gas monitor uses a percutaneous gas sensor in its sensing part of the sensor. The percutaneous gas sensor is affixed to the living body with the sensor affixing part including adhesive tapes and the gas collected from the body surface by sampling is transmitted to the biological information processing part of the detecting part.

The biological information processing part analyzes the transmitted gas components, converts the analysis result to an electrical signal and transmits it as a gas component signal to the mount detecting part. The mount detecting part detects whether or not the sensor communicating with the apparatus is mounted on the living body based on whether or not the specified amount of the gas component of the measurement target exists.

### (Non-invasive blood pressure (NIBP) monitor)

The apparatus for informing about a sensor mounting time period using the NIBP monitor uses a cuff that serves both as the sensing part and the affixing part of the sensor. The pressure and the pressure pulse wave accompanying the cardiac motion are detected inside the cuff wrapped around the patient's arm. The pressure sensor converts the pressure and the pressure pulse wave into electrical signals and transmits them to the biological information processing part of the detecting part.

The biological information processing part extracts the cuff pressure and pressure pulse wave, and transmits them as the cuff pressure signal and the pressure pulse wave signal to the mount detecting part. As to the part that measures the blood pressure in the biological information processing part is similar to that of the conventional NIBP monitor, so that its description is omitted here. The mount detecting part measures the pulse wave signal extracted by the biological information processing part to obtain DC components, amplitudes and intervals, determines whether or not the sensor communicating with the apparatus is mounted on the living body based on whether or not the measured pulse wave signal and cuff pressure values are within the specified ranges. Moreover, the rising times and shapes of the pulse wave signal can be used as additional conditions in the judgment as well.

As can be seen from above, the apparatus for informing about a sensor mounting time period according to the present embodiment detects whether or not the sensor communicating with the apparatus is mounted on the living body, and measures the elapsed time period during which a sensor communicating with the apparatus is mounted continuously on the living body based on the detection result. It then makes a judgment as to whether or not the elapsed time period has reached the specified standard time, and reports that the elapsed time period has reached the standard time base on the detection result.

The present embodiment described in the above provides the following effect.
(a) According to the method and apparatus for informing about a sensor mounting time period according to the present embodiment, the fact that the elapsed time period during which a sensor communicating with the apparatus is mounted continuously on the living body has reached the specified standard time is reported to medical professionals. Therefore, it is possible to prompt the medical professionals to change the mounting position of the sensor to be changed within the specified time. As a result, the burden of the patient associated with the mounting of the sensor can be mitigated.
(b) The sensor herein includes the probe of the pulse oximeter for measuring the arterial blood oxygen saturation. Therefore, it is possible to prompt the medical professionals to change the mounting position of the probe to be changed within the specified time. As a result, the burden of the patient accompanying the mounting of the probe can be reduced.
(c) The apparatus for informing about a sensor mounting time period according to the present embodiment is equipped with the sensor information storing part where the sensor information including the sensor type. The standard time can be set up properly in accordance with the shape or characteristics of each sensor as the standard time can be set in accordance with the type of the sensor.

### (Second embodiment)

In the first embodiment, the standard time setting part is connected to the sensor information storing part and the discriminating part, and the standard time is set in accordance with the type of the sensor.

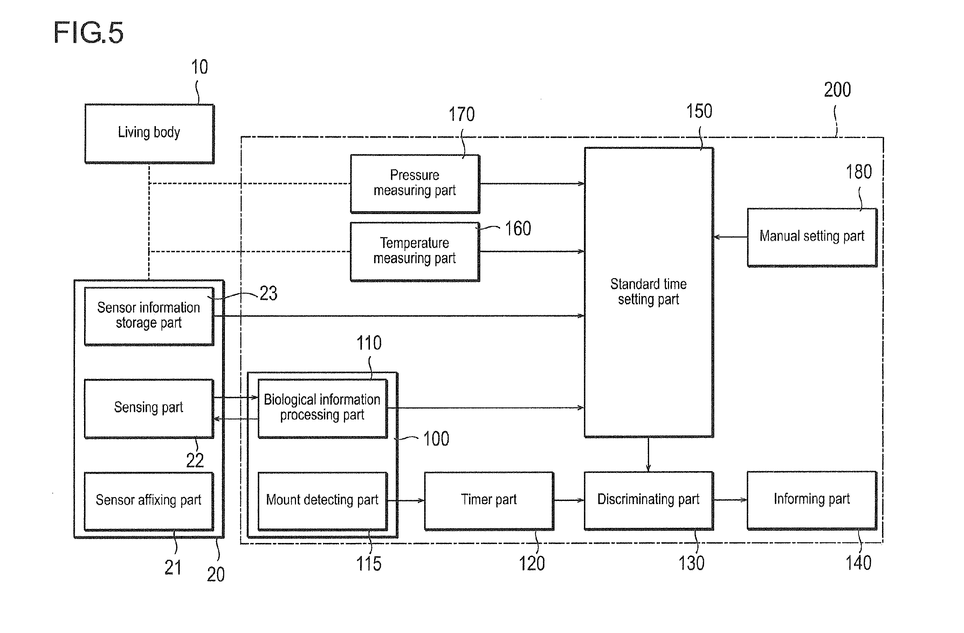

Fig. 5 shows an outline block diagram for describing the apparatus for informing about a sensor mounting time period according to a second embodiment. As shown in Fig. 5, the apparatus 200 for informing about a sensor mounting time period according to the present embodiment further has a temperature measuring part 160, a pressure measuring part 170, and a manual setting part 180 in addition to the configuration of the apparatus 200 for informing about a sensor mounting time period according to the first embodiment.

In the present embodiment, the standard time setting part 150 is further connected to the biological information processing part 110, the temperature measuring part 160, the pressure measuring part 170, and the manual setting part 180 in addition to the sensor information storing part 23 and the discriminating part 130.

The temperature measuring part 160 directly or indirectly measures the temperature of the contact area between the living body 10 and the sensor 20. If it is desired to measure the body temperature directly, it is achieved by causing the sensor 20 to contact with the surface of the living body 10 directly.

On the other hand, if it is difficult to measure the body temperature directly so that it is attempted to measure body temperature indirectly, it is possible to estimate the temperature of the contact area by measuring the temperature related to the contact area. For example, if it is assumed that the thermal gradient from the light-emitting part of the sensor 20 to the body surface does not change depending on the condition, it is possible to estimate the temperature of the body surface by measuring the temperature of the light emitting part. The temperature of the light-emitting part can be estimated by measuring the forward direction voltage in case the light-emitting part is a light-emitting diode.

The pressure measuring part 170 directly or indirectly measures the pressure of the contact area between the living body 10 and the sensor 20. If it is desired to measure the contact pressure of the sensor 20 against the living body 10 directly, it is achieved by causing a pressure sensor to contact on the surface of the living body to measure the pressure of the contact area between the living body 10 and the sensor 20.

On the other hand, if it is attempted to measure the pressure of the sensor 20 against the living body 10 indirectly as it is difficult to measure the pressure of the contact area directly, it is possible to estimate the pressure on the living body, for example, by measuring the strain or elongation of the sensor affixing part 21, etc.

The standard time setting can be done as follows.

In the present embodiment, the standard time can be set in accordance with at least one of the items: the type of the sensor 20, biological information, the temperature of the contact area between the living body 10 and the sensor 20, and the contact pressure between the sensor 20 and the living body 10. The standard time can be set up by medical professionals preferentially and directly in a manual mode.

The standard time can be set up using the measured biological information as follows;

The biological information is transmitted from the biological information processing part 110 to the standard time setting part 150. The standard time setting part 150 can set the standard time based on the biological information processing result received from the biological information processing part 110. For example, even if the same sensor is used, there is a chance that the mounting position may need to be changed earlier than the normal standard depending on the patient's condition.

For example, a baby of a low birth-weight having extremely weak skins, or a patient with a poor peripheral circulation, requires a more frequent change of the mounting position of the sensor 20. The standard time setting part 150 can set up the standard time shorter than usual, if the amplitude of the pulse wave measured by the biological information processing part 110 is extremely small and does not meet the specified value. Also, the standard time setting part 150 can set up the standard time shorter than usual in correspondence with the cardiac rate, if the cardiac rate is extremely high or extremely low.

The standard time can be set up using the measured temperature as follows;

The temperature of the contact area between the living body 10 and the sensor 20 is transmitted as the temperature measuring result from the temperature measuring part 160 to the standard time setting part 150. The standard time setting part 150 can set up the standard time based on the temperature measuring result received from the temperature measuring part 160. Moreover, the standard time setting part 150 can be equipped with the compensation device. The compensation device compensates, for example, in such a way that the higher the measured temperature, the shorter the standard time. Consequently, if the temperature of the contact area between the living body 10 and the sensor 20 is high, the standard time is set shorter. As a result, the burden of the patient on the skin due to the heat of the light-emitting part can be reduced.

Moreover, the standard time setting part 150 can set the standard time between 0 and several seconds if the measured temperature exceeds the specified temperature. As a result, the discriminating part 130 can issue the standard time reaching signal instantaneously. Therefore, if the temperature of the contact area between the living body 10 and the sensor 20 is higher than the specified value, it is possible to take an emergency measure of causing the system to be shut down immediately or to shift its condition safer for the patient.

The standard time can be set up using the measured contact pressure as follows;

The contact pressure between the living body 10 and the sensor 20 is transmitted as the contact pressure result from the pressure measuring part 170 to the standard time setting part 150. The standard time setting part 150 can set up the standard time based on the pressure measuring result received from the pressure measuring part 170. Moreover, the standard time setting part 150 can be equipped with the compensation device. The compensation device compensates, for example, in such a way that the higher the measured pressure, the shorter the standard time. Consequently, if the contact pressure between the living body 10 and the sensor 20 is high, the standard time is set shorter. As a result, it is possible to prevent an excessive contact pressure from being applied to the patient's skin by the sensor 20 for a prolonged period of time.

Moreover, the standard time setting part 150 can set the standard time between 0 and several seconds if the measured contact pressure exceeds the specified value. As a result, the discriminating part 130 can issue the standard time reaching signal instantaneously. Therefore, if the contact pressure between the living body 10 and the sensor 20 is higher than the specified value, it is possible to take an emergency measure of causing the system to be shut down immediately or to shift its condition safer for the patient.

The standard time can be set up directly by a medical professional in a manual mode as follows:

The standard time setting part 150 can set up the standard time based on the manual setting information received from the manual setting part 180. The manual setting part 180 is equipped with, for example, an input keyboard, a touch panel, etc. The standard time setting part 150 can set up the standard time based on the manual setting information entered by a medical professional via the manual setting part 180. The manual setting information can be information related to the patient's physical condition in addition to the value corresponding to the standard time.

As can be seen from the above, in the present embodiment, the standard time can be set in accordance with at least one of the items: the type of the sensor 20, biological information, the temperature of the contact area between the living body 10 and the sensor 20, and the contact pressure between the sensor 20 and the living body 10. The standard time can be set up by medical professionals preferentially and directly in a manual mode.

As can be seen from the above, the present embodiment provides the following effects in addition to the effects provided by the first embodiment.
(d) The apparatus for informing about a sensor mounting time period according to the present embodiment is equipped with the manual setting part for manually setting the standard time. Therefore, it enables medical professionals to set up the standard time more appropriately in accordance with the patient's physical condition.
(e) The apparatus for informing about a sensor mounting time period according to the present embodiment is equipped with the temperature measuring part for measuring the temperature of the contact area between the living body and the sensor directly or indirectly. Moreover, the standard time can be set up or compensated using the measurement result of the temperature measuring part. Consequently, if the temperature of the contact area between the living body and the sensor is high, the standard time is set shorter. As a result, the burden of the patient on the skin due to the heat of the light-emitting part can be reduced.
(f) The apparatus for informing about a sensor mounting time period according to the present embodiment is equipped with the pressure measuring part for measuring the contact pressure between the living body and the sensor directly or indirectly. Moreover, the standard time can be set up or compensated using the measurement result of the pressure measuring part. Consequently, if the contact pressure between the living body and the sensor is high, the standard time is set shorter. As a result, it is possible to prevent an excessive contact pressure from being applied to the patient's skin by the sensor for a prolonged period of time.

Such is the configuration of the apparatus for informing the sensor mounting time period according to the present embodiment. However, it goes without saying that the present invention can arbitrarily added, modified and omitted by a person skilled in the art within the gist of the technology disclosed herein.

For example, in the embodiments described above, the configuration of the apparatus for informing about a sensor mounting time period is such that the notification is issued using the biological information monitors, e.g., pulse oximeter, electrocardiograph, electroencephalograph, clinical thermometer, phonocardiograph, percutaneous gas monitor, and NIBP monitor. However, the biological information monitor applicable is not limited to the abovementioned biological information monitor, but rather any other types of sensors such as a respiratory sound measuring device, electromyograph, tissue oximeter, etc., can be used to configure the apparatus for informing about a sensor mounting time period so long as they can be affixed on the living body surface.

Moreover, in the embodiments described above, the standard time is adjusted using at least one kind of information from the group; type of the sensor, temperature measurement result, pressure measurement result, biological information measurement result, and manual setup information. However, it is possible to achieve the same effect as adjusting the standard time by adjusting the counting speed by modifying the clock cycle of the digital counter of the timer part while maintaining the standard time constant.

Furthermore, it is possible also to detect the sensor mounting on the living body by measuring the temperature of the contact area between the living body and the sensor and/or the contact pressure between the living body and the sensor. In other words, the mounting of the sensor on the living body can be identified so long as the temperature and/or the pressure falls within the specified ranges.

## Claims

1. A method of informing about a sensor mounting time period by measuring the time period a sensor(20) has been mounted on a living body(10) for the purpose of measuring biological information, and informing when the time period has reached or reaches a specified standard time.

2. An apparatus for informing about a sensor mounting time period, configured for communicating with at least one sensor (20) mounted on a living body for measuring biological information, said apparatus (200) comprising:
a detecting part(115) for detecting whether or not any sensor(20) communicating with said apparatus is mounted on the living body(10) for measuring biological information;
a timer part(120) for counting an elapsed time period during which the sensor(20) is continuously mounted on the living body(10) based on the detection result of the detecting part(120);
a discriminating part(130) for discriminating whether or not the elapsed time has reached the specified standard time; and
an informing part(140) for issuing a notification when the elapsed time has reached the standard time based on the discrimination result of the discriminating part(130).

3. The apparatus for informing about a sensor mounting time period as claimed in Claim 2 further comprising:
a sensor information storing part(23) for storing the sensor information including sensor types, wherein
the standard time is set up in accordance with the sensor type.

4. The apparatus for informing about a sensor mounting time period as claimed in Claim 2 or Claim 3 further comprising:
a manual setting part(180) for manually setting the standard time.

5. The apparatus for informing about a sensor mounting time period as claimed in any of Claims 2 to 4 further comprising:
a temperature measuring part(160) for directly or indirectly measuring temperature of a contact area between the living body(10) and the sensor(20), wherein
the standard time is set up using the measurement result of the temperature measuring part(160).

6. The apparatus for informing about a sensor mounting time period as claimed in any of Claims 2 to 5 further comprising:
a pressure measuring part(170) for directly or indirectly measuring pressure of a contact area between the living body(10) and the sensor(20), wherein
the standard time is set up using the measurement result of the pressure measuring part(170).

7. The apparatus for informing about a sensor mounting time period as claimed in any of Claims 2 to 6 further comprising:
the sensor(20) includes a probe of a pulse oximeter for measuring the arterial blood oxygen saturation.

8. Use of the apparatus (200) as claimed in any of claims 2 to 7 for informing about a time period during which a sensor (20) has been mounted on a living body for measuring biological information.

9. The use as claimed in claim 8, wherein the sensor(20) includes a probe of a pulse oximeter for measuring the arterial blood oxygen saturation of said living body.
